# EUROPEAN PATENT SPECIFICATION

(11) **EP 1 319 020 B1**
(45) Date of publication and mention of the grant of the patent: **09.05.2007**
(21) Application number: 01980370.9
(22) Date of filing: 11.09.2001
(51) Int. Cl.: C07K 14/415

(54) **VARIANTS OF THE MAJOR ALLERGEN Par j 2 OF Paritaria judaica**
VARIANTEN DES HAUPTALLERGEN PAR J 2 VON PARIETARIA JUDAICA
ALLELES DE L'ALLERGENE MAJEUR PAR J 2 DE PARIETARIA JUDAICA

(30) Priority: 12.09.2000 IT MI001985
(43) Date of publication of application: 18.06.2003
(73) Proprietor: CONSIGLIO NAZIONALE DELLE RICERCHE, 00185 Roma (IT)
(72) Inventor: STURARO, Monica, I-00185 Roma (IT); VIOTTI, Angelo, I-00185 Roma (IT); GENGA, Annamaria, I-00185 Roma (IT); FALAGIANI, Paolo, I-20143 Milano (IT); MISTRELLO, Giovanni, I-20143 Milano (IT); RONCAROLO, Daniela, I-20143 Milano (IT); ZANOTTA, Stefania, I-20143 Milano (IT)
(74) Representative: Minoja, Fabrizio
(86) International application number: PCT/EP2001/010483
(87) International publication number: WO 2002/022674

(56) References cited:
- EP-A- 0 707 065
- WO-A-00/47610
- WO-A-00/52154
- WO-A-99/34826
- WO-A-99/47680
- DURO G. ET AL.: "cDNA cloning, sequence analysis and allergological characterization of Par i 2.0101, a new major alergen of the Parietaria judaica pollen." FEBS LETTERS, vol. 399, - 1996 pages 295-298, XP002191113
- COLOMBO P ET AL: "Identification of an immunodominant IgE epitope of the Parietaria judaica major allergen" JOURNAL OF IMMUNOLOGY, THE WILLIAMS AND WILKINS CO. BALTIMORE, US, vol. 160, no. 6, 15 March 1998 (1998-03-15), pages 2780-2785, XP002186915 ISSN: 0022-1767
- COSTA M A ET AL.: "The IgE-binding epitopes of rPar j 2, a major allergen of Parietaria judaica pollen, are heterogeneously recognized among allergic subjects." ALLERGY, vol. 55, no. 3, 2000, pages 246-250, XP001055430
- FERREIRA F ET AL: "Modulation of IgE reactivity of allergens by site-directed mutagenesis: potential use of hypoallergenic variants for immunotherapy" FASEB JOURNAL, FED. OF AMERICAN SOC. FOR EXPERIMENTAL BIOLOGY, BETHESDA, MD, US, vol. 12, no. 2, 1 February 1998 (1998-02-01), pages 231-242, XP002085249 ISSN: 0892-6638
- BONURA A ET AL: "HYPOALLERGENIC VARIANTS OF THE PARIETARIA JUDAICA MAJOR ALLERGEN PAR J 1: A MEMBER OF THE NON-SPECIFIC LIPID TRANSFER PROTEIN PLANT FAMILY" INTERNATIONAL ARCHIVES OF ALLERGY AND IMMUNOLOGY, XX, XX, vol. 126, no. 1, September 2001 (2001-09), pages 32-40, XP001037388 ISSN: 1018-2438

## Description

The present invention relates to novel variants of an allergen of the pollen of plants of the species Parietaria judaica.

More particularly, the present invention relates to the amino acidic sequences of hypoallergenic variants of the allergen Par j 2, obtained by site-specific mutagenesis of the nucleotidic sequence encoding said allergen. The hypoallergenic variants can be used in the specific immunotherapy of allergic pathologies caused by Parietaria judaica pollen.

### BACKGROUND OF THE INVENTION

Allergies are caused by a disfunction of the immune system, which reacts producing antibodies of the class IgE against proteins, mainly contained in pollens, mites, epithelia, and some foodstuff, which proteins are per se innocuous.

Recent estimations indicate that above 10% of population in Western countries suffers from this disease, which in time may induce worsening of symptoms (e.g. appearance of asthma) and sensitization to other allergens, thus making the choice of therapy more complicated.

Specific immunotherapy (SIT), contrary to pharmacological therapy, is the only kind of etiological treatment of allergic pathologies capable of favourably affecting some immunological parameters which are at the basis of the disease.

SIT consists in the administration of increasing doses of standardized extracts (vaccines), obtained starting from the substance which is the cause of the disease.

This way, immunological tolerance to said substance gradually increases in the patient, accompanied by disappearance of the allergic symptoms.

The risk of eliciting even serious side effects (1), although remarkably reduced with the use of either slow-release vaccines or vaccines administered through alternative route to the injective one, has however restricted the use of SIT in the therapy of allergic diseases.

In recent years, attention has been focused on the development of effective, safer vaccines. In particular, an important target is the development of vaccines consisting of mutagenized recombinant proteins, i.e. hypoallergenic variants capable of favourably affecting the natural progress of the disease without causing undesired side effects (2).

Parietaria pollen is one of the most important causes of allergy in the Mediterranean area. The two main allergens of this pollen, Par j 1 (whose nucleotide sequence is identified in GenBank under the accession code AC X77414) and Par j 2 (AC X95865), are proteins of approximately 12 kD molecular weight with sequence and functional partial homology (3, 4, 5).

### Detailed disclosure of the invention

It now has been found that the allergenic effect of Par j 2 (GenBank, AC X95865) may be decreased by changing its amino acid sequence in at least one of the positions n. 19, 23, 27, 35, 41, 46, 73 and 78, in which a Lys residue is present. "Change" herein means substituting one or more residues in the specified positions preferably with neutral or polar amino acids, or deleting one or more Lys residues present in the natural form, or simultaneously substituting and deleting two or more residues.

The following mutations by substitution, are preferred: Gln19, Ala23, Ala27, Gly35, Ala41, Ala46, Gly73 and Ser78, wherein the number means the position of the amino acidic residue in the sequence. More preferred are the variants in which the eight substitutions indicated above (SEQ ID N. 2) or, alternatively, the 5 substitutions Gln19, Ala23, Ala27, Ala41 and Ala46 (SEQ ID N. 3), are at the same time present.

The invention further comprises an immunologically active peptide deriving from the amino acidic sequence of Par j 2 and containing at least one of the substitutions/deletions described above.

In a further aspect, the invention is directed to a nucleic acid molecule encoding for a protein variant of Par j 2 or for a peptide derived therefrom.

The sequence variants according to the invention can easily be prepared starting from cDNA of the allergen Par j 2 mature form, which does not include the region coding the signal peptide, suitably mutagenized at the desired positions.

The cDNA sequence coding the preferred variant corresponding to SEQ ID N. 2, is reported in SEQ ID N. 1.

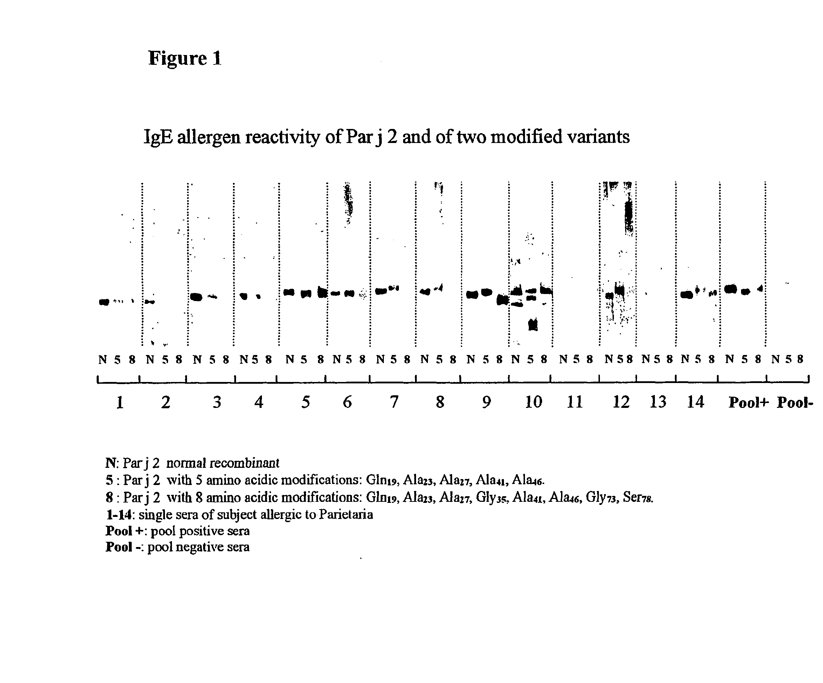

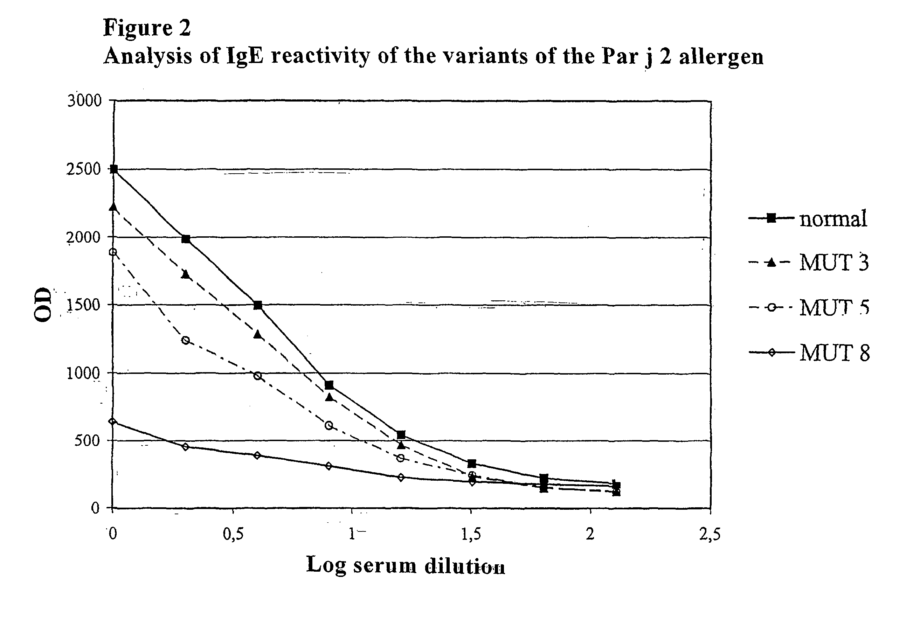

The cDNA of SEQ ID N. 1 was expressed in Escherichia coli cells. The produced recombinant protein has reduced reactivity to IgEs of serum from subjects allergic to Parietaria judaica pollen. In particular, Western blotting tests proved that approximately 75% (10/13) of sera immunoreacting with the normal allergen have reduced reactivity with the mutagenized variant corresponding to SEQ ID N. 2 [Fig. 1]. This reactivity decreases by more than 80% on the average, as determined by ELISA assay [Fig. 2, mut 8]. On the other hand, the variant obtained by introducing the residues Gln19, Ala23, Ala27, Ala41 and Ala46 showed reduced allergenicity in half the tested sera [Fig. 1] and said reduction is 50% on the average [Fig. 2, mut 5]. Finally, the variant of the allergen Par j 2 with the 3 substitutions Gln19, Ala23 and Ala27 shows reduction of the IgE binding of about 20% [Fig. 2, mut 3].

The invention further relates to an expression vector comprising a nucleic acid molecule coding for any one of the hypoallergenic variants defined above.

Said vector can be a plasmid, cosmid, virus, bacteriophage or any other vector commonly used in genetic engineering, and can include, in addition to the nucleic acid molecule of the invention, eukaryotic or prokaryotic elements for the control of the expression, such as regulatory sequences for the initiation and the termination of the transcription, enhancers, promoters, signal sequences and the like.

Moreover, the invention comprises a prokaryotic or eukaryotic host cell transformed into or transfected with the vector of the invention. In principle, prokaryotic cells such as Escherichia coli or Bacillus subtilis, or eukaryotic cells such as Saccharomyces cerevisiae will be used for cloning the vector and expressing the cDNA.

The protein variants of the invention can be produced either as such or as fusion proteins.

Thanks to the reduced IgE reactivity, said variants may be used for therapeutical purposes in the preparation of vaccines to be used in the immunotherapy of allergies to Parietaria judaica pollen.

A further aspect of the invention relates therefore to a pharmaceutical composition comprising an effective amount of the hypoallergenic variant of the invention, optionally in combination with other natural or modified allergens of Parietaria judaica, together with pharmaceutically acceptable excipients.

In a preferred embodiment, said pharmaceutical composition is a vaccine for use in the prophylactic or therapeutical treatment of allergic diseases, such as bronchial asthma, allergic rhinitis, allergic dermatitis, allergic conjunctivitis. Vaccination principles and practice are well known to those skilled in the art and are described, for example, in (7) and (8).

The following examples illustrate the invention in greater detail.

The methods used in the following examples, if not otherwise specified, are those described by Sambrook, Fritsch ET Maniatis "Molecular cloning. A laboratory manual" II Ed. vol. 1-2-3 CSH Lab Press 1989.

### Example 1 - Site-specific mutagenesis of the cDNA coding for the allergen Par j 2

The site-specific mutagenesis of the cDNA coding for the allergen Par j 2 is carried out by PCR amplification (Polymerase Chain Reaction) of the same cDNA cloned in a prokaryotic vector (pBluescript).

The oligonucleotides used as primer for the PCR reaction have the required substitutions of bases. For each mutagenesis, a complementary pair of said oligonucleotides has been used, which bind to corresponding regions of the two DNA strands. After amplification, the original, unchanged template is selectively degraded by enzymatic digestion catalyzed by the restriction enzyme Dpn I. Escherichia coli cells are then transformed with the mutagenized molecules. Clones obtained from single bacterial colonies are sequenced according to the Sanger method to verify the correct modification of the bases and the absence of cDNA aspecific mutations.

### Example 2 - Production of the protein Par j 2 and of the variants thereof

Normal cDNA from Par j 2 and mutagenized cDNA, after cloning in an expression vector (pCALn - Stratagene), are expressed in Escherichia coli cells according to standard protocols. Cells are collected by centrifugation and resuspended in PBS buffer. The recombinant proteins are isolated after lysis of the bacterial cells by sonication and removal of cell particulates by centrifugation. Proteins are purified from supernatant by affinity chromatography, using columns wherein the matrix is bonded to the calmodulin protein, which interacts with the CBP portion (Calmodulin Binding Protein) fused to the allergen.

### Example 3 - Western blotting assay of the allergenicity of the Par j 2 variants

Equal amounts of the recombinant allergen and of the mutagenized variant are analyzed by electrophoresis on polyacrylamide gel and subsequent transfer onto nitro-cellulose membrane by electroblotting, according to the technique described by Towbin (6).

The membrane is incubated for an hour in TBS containing 5% of powder milk (saturation buffer) then overnight with single sera from patients allergic to Parietaria (RAST 3+ and 4+). After three washings with TBS containing 0.05% Tween-20, IgE antibodies bound to the membrane are detected by incubation for an hour with anti-human IgE peroxidase-conjugated antiserum and, after further washings, with the detection system based on the use of a DAB (diaminobenzidine) solution containing H₂O₂ as substrate for the peroxidase.

### Example 4 - ELISA assay for the reactivity to IgE of the Par j 2 variants

Equal amounts (0.2 µg) of normal allergen and of its mutagenized variants, in carbonate/bicarbonate 50 mM buffer, pH 9,6, are adsorbed on wells of polystyrene plates for ELISA tests by incubation at 4°C for 16 hours. The antigens are then washed with washing solution (60 mM phosphate buffer pH 6,5 containing 0.05% Tween-20) and the free sites are saturated with diluent solution (25% horse serum, EDTA 1 mM, 0.05% Tween 20, 0.01% Thiomersal in phosphate buffer 150 mM pH 7,4). Serial dilutions of human serum pools with RAST 4+ reactivity are prepared in a 1:2 ratio in diluent buffer. Equal amounts (100 µl) of the various serum dilutions are added to each sample and incubated at 25°C for 2 hours. After three washings, the anti-human IgE peroxidase conjugated antiserum diluted 1:1500 in diluent buffer is added, and incubated at 25°C for 1.5 hours. After three washings, the colorimetric reaction is developed by addition of 100 µl of Ultra Blu reagent (Intergen, Milford, MA) and incubation for 15 minutes at 25°C. The reaction is stopped by addition of 100 µl of 1N HCl and evaluated at 450 nm with a spectrophotometer.

### REFERENCES

1) Toubi E., Kessel A., Blant A., Golan T.D., (1999) "Follow-up after systemic adverse reactions of immunotherapy".
   Allergy, 54(6): 617-620
2) Akdis C.A., Blaser K., (2000) "Regulation of specific immune response by chemical and structural modifications of allergens".
   Int. Arch. Allergy Immmunol., 121(4): 261-269
3) Costa M.A., Colombo P., Izzo V., Kennedy D., Venturella S., Cocchiara R., Mistrello G., Falagiani P., Geraci D., (1994). "cDNA cloning, expression and primary structure of Par j I, a major allergen of Parietaria judaica pollen".
   FEBS Lett., 341:182-186
4) Duro G., Colombo P., Costa M.A., Izzo V., Porcasi R., of Fiore R., Locorotondo G., Mirisola M.G., Cocchiara R., Geraci D., (1996). "cDNA cloning, sequence analysis and allergological characterization of Par j 2.0101, a new major allergen of the Parietaria judaica pollen".
   FEBS Lett., 399: 295-298
5) Colombo P., Kennedy D., Ramsdale T., Costa M.A., Duro G., Izzo V., Salvadori S., Guerrini R., Cocchiara R., Mirisola M.G., Wood S., Geraci D., (1998). "Identification of an immunodominant IgE epitope of the Parietaria judaica major allergen".
   J. Immunol., 160: 2780-2785
6) Towbin J., Staehelin T., Gordon J., (1979). "Electrophoretic transfer of proteins from polyacrylamide gels to nitrocellulose sheets: procedures and some applications".
   Proc. Natl. Acad. Sci. USA, 76: 4350-4354
7) Paul, (1989), "Fundamental Immunology", Raven press, New York.
8) Cryz, S. J. (1991), "Immunotherapy and Vaccines", VCH Verlagsgesellschaft.

## Claims

1. A Par j 2 protein having sequence SEQ ID No. 2, in which a Lys residue is present in the positions 19, 23, 27, 35, 41, 46, 73 and 78, wherein at least one of said Lys residues is substituted and/or deleted, said protein having reduced allergenic effect.

2. The protein as claimed in claim 1, wherein said residues are substituted with neutral or polar amino acids.

3. The protein as claimed in claims 1-2, wherein said substitutions are selected from Gln19, Ala23, Ala27, Gly35, Ala41, Ala46, Gly73 and Ser78.

4. The protein as claimed in claims 1-3, having sequence SEQ ID N. 2.

5. The protein as claimed in claims 1-3, bearing the substitutions Gln19, Ala23, Ala27, Ala41 and Ala46 (SEQ ID N. 3).

6. A peptide comprising an immunologically active part of the protein of claims 1-5, wherein at least one substitution/deletion as claimed in claim 1 is present.

7. A nucleic acid molecule coding for a protein as claimed in claims 1-5 or for a peptide as claimed in claim 6.

8. A nucleic acid molecule as claimed in claim 7, of sequence SEQ ID N. 1.

9. A vector comprising the nucleic acid molecule of claims 7-8.

10. A host cell transduced with the vector of claim 9.

11. A pharmaceutical composition comprising an effective amount of a protein as claimed in claims 1-5 or of a peptide as claimed in claim 6 together with pharmaceutically acceptable excipients.

12. A composition as claimed in claim 11, in the form of a vaccine.

## Patentansprüche

1. Ein Par j 2 Protein mit Sequenz SEQ ID NO. 2, in welcher ein Lys-Rest an den Positionen 19, 23, 27, 35, 41, 46, 73 und 78 vorhanden ist, wobei wenigstens einer der besagten Lys-Reste substituiert und/oder deletiert ist und besagtes Protein einen erniedrigten allergenen Effekt hat.

2. Das Protein wie in Anspruch 1 beansprucht, wobei besagte Reste durch neutrale oder polare Aminosäuren substituiert sind.

3. Das Protein wie in den Ansprüchen 1-2 beansprucht, wobei besagte Substitutionen ausgewählt sind aus Gln19, Ala23, Ala27, Gly35, Ala41, Ala46, Gly73 und Ser78.

4. Das Protein wie in den Ansprüchen 1-3 beansprucht, mit der Sequenz SEQ ID NO. 2.

5. Das Protein wie in den Ansprüchen 1-3 beansprucht, mit den Substitutionen Gln19, Ala23, Ala27, Ala41 and Ala 46 (SEQ ID NO. 3).

6. Ein Peptid umfassend einen immunologisch aktiven Teil des Proteins gemäß der Ansprüche 1-5, wobei wenigstens eine Substitution/Deletion, wie in Anspruch 1 beansprucht, vorhanden ist.

7. Ein Nukleinsäuremolekül kodierend für ein Protein wie in den Ansprüchen 1-5 beansprucht, oder ein Peptid, wie in Anspruch 6 beansprucht.

8. Ein Nukleinsäuremolekül wie in Anspruch 7 beansprucht, mit der Sequenz SEQ ID NO. 1.

9. Ein Vektor umfassend das Nukleinsäuremolekül gemäß der Ansprüche 7-8.

10. Eine Wirtszelle, transduziert mit dem Vektor gemäß Anspruch 9.

11. Eine pharmazeutische Zusammensetzung umfassend eine wirksame Menge eines Proteins wie in den Ansprüchen 1-5 beansprucht, oder eines Peptids wie in Anspruch 6 beansprucht zusammen mit pharmazeutisch verträglichen Trägerstoffen.

12. Eine Zusammensetzung wie in Anspruch 11 beansprucht in der Form eines Impfstoffs.

## Revendications

1. Protéine Par j 2 ayant la séquence SEQ ID No.2, dans laquelle un résidu Lys est présent aux positions 19, 23, 27, 35, 41, 46, 73 et 78, dans laquelle au moins un desdits résidus Lys est substitué et/ou supprimé, ladite protéine ayant un effet allergène réduit.

2. Protéine selon la revendication 1, dans laquelle lesdits résidus sont substitués avec des acides aminés neutres ou polaires.

3. Protéine selon les revendications 1-2, dans laquelle lesdites substitutions sont choisies parmi Gln19, Ala23, Ala27, Gly35, Ala41, Ala46, Gly73 et Ser78.

4. Protéine selon les revendications 1-3, ayant la séquence SEQ ID No.2.

5. Protéine selon les revendications 1-3, portant les substitutions Gln19, Ala23, Ala27, Ala41 et Ala46 (SEQ ID No. 3).

6. Peptide comprenant une partie immunologiquement active de la protéine selon les revendications 1-5, dans lequel au moins une substitution/suppression selon la revendication 1 est présente.

7. Molécule d'acide nucléique codant pour une protéine selon les revendications 1-5 ou pour un peptide selon la revendication 6.

8. Molécule d'acide nucléique selon la revendication 7, de séquence SEQ ID No.1.

9. Vecteur comprenant la molécule d'acide nucléique selon les revendications 7-8.

10. Cellule-hôte transduite avec le vecteur selon la revendication 9.

11. Composition pharmaceutique comprenant une quantité efficace d'une protéine selon les revendications 1-5 ou d'un peptide selon la revendication 6, ainsi que des excipients acceptables sur le plan pharmaceutique.

12. Composition selon la revendication 11, sous la forme d'un vaccin.


### SEQUENCE LISTING

<110> CONSIGLIO NAZIONALE DELLE RICERCHE
<120> VARIANTS OF THE MAJOR ALLERGEN PAR J 2 OF PARIETARIA JUDAICA
<130> cnr
<140>
   <141>
<160> 3
<170> PatentIn Ver. 2.1
<210> 1
   <211> 306
   <212> DNA
   <213> Parietaria judaica
<400> 1
   gaggaggctt gcgggaaagt ggtgcaggat ataatgccgt gcctgcattt cgtgcagggg 60
   gaggaggcgg agccgtcggc ggagtgctgc agcggcacga aggggctgag cgaggaggtg 120
   gcgacgacgg agcaggcgag ggaggcctgc aagtgcatag tgcgcgccac gaagggcatc 180
   tccggtatca aaaatgaact tgtcgccgag gtccccggga agtgcgatat tagcaccact 240
   ctcccgccca tcaccgccga cttcgactgc tccaagatcc aaagtactat tttcagaggt 300
   tactat                                                            306
<210> 2
   <211> 102
   <212> PRT
   <213> Parietaria judaica
<400> 2
   Glu Glu Ala Cys Gly Lys Val Val Gln Asp Ile Met Pro Cys Leu His
   1               5                   10                  15
   Phe Val Gln Gly Glu Glu Ala Glu Pro Ser Ala Glu Cys Cys Ser Gly
               20                  25                  30
   Thr Lys Gly Leu Ser Glu Glu Val Ala Thr Thr Glu Gln Ala Arg Glu
           35                  40                  45
   Ala Cys Lys Cys Ile Val Arg Ala Thr Lys Gly Ile Ser Gly Ile Lys
       50                  55                  60
   Asn Glu Leu Val Ala Glu Val Pro Gly Lys Cys Asp Ile Ser Thr Thr
   65                  70                  75                  80
   Leu Pro Pro Ile Thr Ala Asp Phe Asp Cys Ser Lys Ile Gln Ser Thr
                   85                  90                  95
   Ile Phe Arg Gly Tyr Tyr
               100
<210> 3
   <211> 102
   <212> PRT
   <213> Parietaria judaica
<400> 3
   Glu Glu Ala Cys Gly Lys Val Val Gln Asp Ile Met Pro Cys Leu His
    1              5                  10                  15
   Phe Val Gln Gly Glu Glu Ala Glu Pro Ser Ala Glu Cys Cys Ser Gly
               20                  25                  30
   Thr Lys Lys Leu Ser Glu Glu Val Ala Thr Thr Glu Gln Ala Arg Glu
           35                  40                  45
   Ala Cys Lys Cys Ile Val Arg Ala Thr Lys Gly Ile Ser Gly Ile Lys
       50                  55                  60
   Asn Glu Leu Val Ala Glu Val Pro Lys Lys Cys Asp Ile Lys Thr Thr
   65                  70                  75                  80
   Leu Pro Pro Ile Thr Ala Asp Phe Asp Cys Ser Lys Ile Gln Ser Thr
                   85                  90                  95
   Ile Phe Arg Gly Tyr Tyr
               100